(19) **Europäisches Patentamt / European Patent Office / Office européen des brevets**

(11) **EP 4 410 324 A1**

(12) # DEMANDE DE BREVET EUROPEEN

(43) Date de publication:
**07.08.2024 Bulletin 2024/32**

(21) Numéro de dépôt: **24155952.5**

(22) Date de dépôt: **06.02.2024**

(51) Classification Internationale des Brevets (IPC):
**A61L 27/36** (2006.01)     **A61L 27/50** (2006.01)

(52) Classification Coopérative des Brevets (CPC):
**A61L 27/3604; A61L 27/3645; A61L 27/3662; A61L 27/3679; A61L 27/50;** A61L 2430/06; A61L 2430/10; A61L 2430/22; A61L 2430/30; A61L 2430/34

(84) Etats contractants désignés:
**AL AT BE BG CH CY CZ DE DK EE ES FI FR GB GR HR HU IE IS IT LI LT LU LV MC ME MK MT NL NO PL PT RO RS SE SI SK SM TR**
Etats d'extension désignés:
**BA**
Etats de validation désignés:
**GE KH MA MD TN**

(30) Priorité: **06.02.2023 FR 2301113**
**15.01.2024 FR 2400351**

(71) Demandeur: **TBF Genie Tissulaire (TBF)**
**69780 Mions (FR)**

(72) Inventeur: **BARNOUIN, Laurence**
**38460 VENERIEU (FR)**

(74) Mandataire: **Tripoz, Inès**
**Cabinet Tripoz**
**Le Pôle Sud**
**22 rue Seguin**
**69002 Lyon (FR)**

(54) ## STRUCTURE PLACENTAIRE ET/OU OMBILICALE DESSIQUÉE

(57)     La présente invention concerne le domaine de l'ingénierie tissulaire, plus particulièrement elle concerne une structure placentaire et/ou ombilicale obtenue par au moins une étape de séchage par par dessication notamment utilisable dans des applications en chirurgie orthopédique, en particulier en tant que structure de renfort des articulations ainsi que son procédé d'obtention.

FIGURE 2

EP 4 410 324 A1

**Description**

La présente invention concerne le domaine de l'ingénierie tissulaire, plus particulièrement elle concerne une structure placentaire et/ou ombilicale obtenue par au moins une étape de séchage par dessication notamment utilisable dans des applications en chirurgie orthopédique, en particulier en tant que structure de renfort des articulations ainsi que son procédé d'obtention.

On connait notamment l'utilisation des structures placentaire ou ombilicale lyophilisée pour le traitement des lésions des surfaces oculaires, cutanées ou des ulcères, en raison de leur potentiel électrostatique permettant d'épouser et d'adhérer rapidement à la surface sur laquelle elle est appliquée.

La demande de brevet WO2017/140914 déposée au nom de la demanderesse divulgue notamment un procédé de préparation d'un matériau d'allogreffe obtenu à partir de tissus placentaires issus de mammifère, ledit procédé comprenant la lyophilisation desdits tissus placentaires.

De telles structures sont notamment commercialisées sous la dénomination Membranes VISIO AMTRIX® par la demanderesse.

Les caractéristiques de ce produit qui constituent un avantage dans le cadre traitement des lésions des surfaces oculaires, cutanées ou des ulcères, s'avèrent pouvoir être un inconvénient dans le cadre d'autres types de chirurgie du fait que la structure épouse et adhère également rapidement à la surface de l'instrument ou des gants du praticien avant l'implantation, rendant très complexe toute mise en forme extemporanée. Le repositionnement de la structure in vivo est également rendu difficile pour les mêmes raisons.

Des opérations de mise en forme sont cependant requises pour les adapter à la taille et la forme de la structure aux articulations à traiter dans le cadre de leurs utilisations dans des applications orthopédiques.

Elles doivent en outre pouvoir être glissées entre deux plans de tissus sans adhérer aux éléments constitutifs de celle-ci avant d'atteindre la localisation ciblée ou enroulé autour d'un tendon ou d'un ligament. La capacité de la structure à être repositionnée facilement est un avantage certain pour le praticien.

Lorsque ces structures sont séchées à l'air libre, c'est-à-dire sans application de température ou de pression contrôlées, elles deviennent très rigides et cassantes et doivent être réhydratées pour retrouver de leur souplesse. De plus, ce type de séchage est peu reproductible et va dépendre de nombreux facteurs tels que la température, l'humidité ou la pression atmosphérique du lieu de séchage, mais également des dimensions de la structure à sécher.

De manière tout à fait surprenante, la demanderesse a mis au point un procédé mettant en oeuvre une étape de séchage par dessication des structures placentaires et/ou ombilicales permettant de résoudre l'ensemble des problèmes de l'art antérieur.

Les structures placentaires et/ou ombilicales séchées par au moins une étape de dessication sont en effet plus épaisses, présentent un aspect plus lisse et sont plus facilement manipulables que les structures placentaires et/ou ombilicales séchées par d'autres techniques. Elles sont en effet plus flexibles, plus souples, moins cassantes, n'adhèrent pas aux surfaces biologiques et peuvent être repositionnées après application, notamment au contact d'un fluide biologique, notamment du sang.

Elles présentent en outre l'avantage de pouvoir être utilisées sans étape de réhydratation préalable.

L'étape de séchage par dessication présente par ailleurs l'avantage d'être reproductible précisément indépendamment des dimensions de la structure visée et des conditions extérieures.

Les structures placentaires et/ou ombilicales selon la présente invention sont donc particulièrement adaptées à des applications chirurgicales notamment en tant que structure de maintien et/ou de renfort notamment dans des applications orthopédiques.

La Figure 1 illustre une comparaison structure placentaire consistant en une couche membranaire séchée par dessication (image A), par lyophilisation (image B), ou par séchage à l'air libre (image C).

La Figure 2 illustre une structure placentaire consistant en une couche membranaire séchée par dessication et pliée sur elle-même le long de sa diagonale.

La Figure 3 illustre structure placentaire consistant en une couche membranaire séchée par lyophilisation et pliée sur elle-même le long de sa diagonale.

La présente invention a pour objet une structure placentaire et/ou ombilicale caractérisée en ce qu'elle est séchée par au moins une étape de séchage par dessication.

Au sens de la présente invention, l'expression « au moins un(e) » signifie « un(e) ou plusieurs », « le ou les » ou « la ou les » et pourra être remplacée par ces dernières sans distinction.

La structure placentaire et/ou ombilicale selon la présente invention est obtenue après l'accouchement d'un donneur proprement informé et consentant en accord avec exigences des directives Européennes et la Loi Bioéthique. Cet accouchement peut s'être déroulé par césarienne ou par voie basse.

Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, la structure placentaire et/ou ombilicale selon l'invention est caractérisée en ce qu'elle est d'origine animale ou humaine.

Dans un mode de réalisation, la structure placentaire et/ou ombilicale selon l'invention est caractérisée en ce qu'elle est d'origine humaine.

Dans un mode de réalisation, la structure placentaire et/ou ombilicale est préparée selon le protocole tel que décrit dans la demande WO2017140914A1.

Dans un mode de réalisation, la structure placentaire et/ou ombilicale est utilisable en chirurgie orthopédique.

Dans un mode de réalisation, la structure placentaire et/ou ombilicale est utilisable en chirurgie orthopédique en tant que structure de renfort des articulations

Dans un mode de réalisation, la structure placentaire et/ou ombilicale selon l'invention est caractérisée en ce que la structure placentaire comprend tout ou partie du placenta.

Dans un mode de réalisation, la structure placentaire et/ou ombilicale selon l'invention est comprend un tissu placentaire.

Dans un mode de réalisation, la structure placentaire et/ou ombilicale selon l'invention est caractérisée en ce que la structure placentaire comprend tout ou partie d'au moins une couche membranaire issue de tissue placentaire.

Dans un mode de réalisation, au moins une couche membranaire issue de tissus placentaires comprend de la membrane amniotique, de la membrane choriale, de la membrane tissulaire spongieuse et/ou de la membrane chorioamniotique, chacun de ces feuillets pouvant inclure, ou non, les sous-structures physiologiques constitutives de l'amnios et/ou du chorion.

Dans un mode de réalisation, au moins une couche membranaire issue de tissus placentaires est d'origine animale ou humaine.

Dans un mode de réalisation, au moins une couche membranaire issue de tissus placentaires est d'origine humaine.

Dans un mode de réalisation, au moins une couche membranaire issue de tissus placentaires comprend tout ou partie de la membrane amniotique et/ou tout ou partie de la membrane choriale.

Dans un mode de réalisation, au moins une couche membranaire issue de tissus placentaires comprend tout ou partie de la membrane amniotique.

Dans un mode de réalisation, la couche membranaire issue de tissus placentaires est une membrane amniotique humaine.

Au sens de la présente invention, on entend par « membrane amniotique », ou « amnios » l'enveloppe tissulaire qui se développe autour de l'embryon, puis du foetus, chez le mammifère durant la grossesse. Elle a pour rôle de protéger l'organisme en développement en maintenant autour de lui le liquide amniotique. Elle est accolée à la deuxième membrane qui est le chorion.

Dans un mode de réalisation, au moins une couche membranaire issue de tissus placentaires comprend au moins une des sous-couches physiologiques de la membrane amniotique choisies dans le groupe comprenant la couche cellulaire épithéliale, la membrane basale, la couche compacte, la couche fibroblastique et/ou la couche spongieuse.

Dans un mode de réalisation préférentiel, au moins une couche membranaire issue de tissus placentaires comprend de la membrane amniotique comprenant sa couche spongieuse.

Dans un mode de réalisation, au moins une couche membranaire issue de tissus placentaires est constituée de la membrane amniotique comprenant sa couche spongieuse.

Dans un mode de réalisation, au moins une couche membranaire issue de tissus placentaires comprend tout ou partie de la membrane choriale.

Au sens de la présente invention, on entend par « membrane choriale », ou « chorion » l'enveloppe tissulaire située entre l'amnios et la paroi utérine qui se développe autour de l'embryon, puis du foetus, chez le mammifère durant la grossesse.

Dans un mode de réalisation, au moins une couche membranaire issue de tissus placentaires comprend au moins une les sous-couches physiologiques de la membrane choriale choisi dans le groupe comprenant la couche réticulaire, la membrane basale et/ou la couche cellulaire trophoblastique.

Au sens de la présente invention, on entend par « membrane chorioamniotique » l'ensemble amnios et chorion, qui n'ont pas été séparés naturellement ou artificiellement, ceux-ci étant reliés par des ponts protéiques.

Au sens de la présente invention, on entend par « membrane tissulaire spongieuse », ou « couche spongieuse » une couche située entre l'amnios et le chorion.

Dans un mode de réalisation, la structure placentaire et/ou ombilicale selon l'invention est caractérisée en ce que la structure ombilicale comprend tout ou partie du cordon ombilical.

Dans un mode de réalisation, la structure placentaire et/ou ombilicale selon l'invention est caractérisée en ce que la structure ombilicale comprend de la gelée de Wharton, au moins un vaisseau ombilical et/ou de la membrane amniotique de cordon ombilical.

Dans un mode de réalisation, la structure placentaire et/ou ombilicale selon l'invention est caractérisée en ce que la structure ombilicale comprend de la gelée de Wharton.

Dans un mode de réalisation, la structure placentaire et/ou ombilicale selon l'invention est caractérisée en ce que la structure ombilicale comprend au moins un vaisseau ombilical.

Dans un mode de réalisation, la structure placentaire et/ou ombilicale selon l'invention est caractérisée en ce que la structure ombilicale comprend au moins un vaisseau ombilical choisi dans le groupe comprenant une veine ombilicale et/ou au moins une artère ombilicale.

Dans un mode de réalisation, la structure placentaire et/ou ombilicale selon l'invention est caractérisée en ce que la structure ombilicale comprend de la membrane amniotique de cordon ombilical.

Dans un mode de réalisation, la structure placentaire et/ou ombilicale selon l'invention est caractérisée en ce que la structure ombilicale comprend de la membrane amniotique de cordon ombilical et de la gelée de Wharton.

Au sens de la présente invention, on entend par « dessication » l'élimination de l'eau contenue dans une substance par vaporisation, à l'aide de la chaleur, du vide et/ou d'une matière hygroscopique.

Dans un mode de réalisation, l'étape de séchage par dessication comprend l'application de chaleur, de vide et/ou d'une matière hygroscopique.

Dans un mode de réalisation, l'étape de séchage par dessication comprend l'application d'une part de la chaleur et d'autre part de vide et/ou d'une matière hyg roscopiq ue.

Dans un mode de réalisation, l'étape de séchage par dessication comprend l'application de chaleur et de vide.

Dans un mode de réalisation, l'étape de séchage par dessication ne comprend pas de congélation et/ou de surgélation de la structure placentaire et/ou ombilicale.

Au sens de la présente invention, on entend par « chaleur », une température supérieure ou égale à 0°C (0°C ≤ température). En fonction de la pression les plages de température utilisées sont les plages de température n'entrainant pas la formation de cristaux d'eau.

Ainsi, les dessiccations sous vide en températures négatives, telle que la cryodessiccation plus communément appelée lyophilisation, sont exclues de la définition de la dessication selon la présente invention.

Dans un mode de réalisation, la structure placentaire et/ou ombilicale selon l'invention n'a subi aucune étape de séchage par lyophilisation.

Au sens de la présente invention, on entend par « lyophilisation » une technique visant à dessécher par sublimation un produit préalablement surgelé, Plus précisément, le liquide à ôter du produit est dans un premier temps transformé en solide (glace) par congélation ; puis par une dessiccation primaire, sous vide, le solide est sublimé ; enfin par une dessiccation secondaire, les molécules d'eau à la surface du produit sont extraites par désorption.

Dans un mode de réalisation, la température minimale de l'étape de dessication est choisie de manière à éviter la formation de cristaux d'eau en fonction de la pression appliquée.

Dans un mode de réalisation, l'étape de séchage par dessication est réalisée par montée progressive de la température à partir d'au moins 0°C (0°C ≤ température).

Dans un mode de réalisation, l'étape de séchage par dessication est réalisée par montée progressive de la température à partir d'au moins 2°C (2°C ≤ température).

Dans un mode de réalisation, l'étape de séchage par dessication est réalisée par montée progressive de la température à partir d'au moins 4°C (4°C ≤ température).

Dans un mode de réalisation, l'étape de séchage par dessication est réalisée par montée progressive de la température jusqu'à 35°C.

Dans un mode de réalisation, l'étape de séchage par dessication est réalisée par montée progressive de la température d'au moins 0°C jusqu'à 50°C.

Dans un mode de réalisation, l'étape de séchage par dessication est réalisée par montée progressive de la température d'au moins 2°C jusqu'à 45°C.

Dans un mode de réalisation, l'étape de séchage par dessication est réalisée par montée progressive de la température d'au moins 4°C jusqu'à 40°C.

Dans un mode de réalisation, l'étape de séchage par dessication est réalisée par montée progressive de la température d'au moins 8°C jusqu'à 35°C.

Dans un mode de réalisation, l'étape de séchage par dessication est réalisée par montée progressive de la température d'au moins 0°C jusqu'à 35°C.

Dans un mode de réalisation, l'étape de séchage par dessication est réalisée par montée progressive de la température d'au moins 0°C à 35°C (0°C ≤ température ≤ 35°C).

Dans un mode de réalisation, l'étape de séchage par dessication est réalisée par montée progressive de la température dans un intervalle allant de 4°C à 35°C (4°C ≤ température ≤ 35°C).

Dans un mode de réalisation, l'étape de séchage par dessication est réalisée par montée progressive de la température par paliers de 1 à 10°C (1°C ≤ température ≤ 10°C).

Dans un mode de réalisation, l'étape de séchage par dessication est réalisée par montée progressive de la température par palier de 1 à 10°C.

Dans un mode de réalisation, l'étape de séchage par dessication est réalisée par montée progressive de la température par palier de 2 à 8°C.

Dans un mode de réalisation, l'étape de séchage par dessication est réalisée par montée progressive de la température par palier de 3 à 7°C.

Dans un mode de réalisation, l'étape de séchage par dessication est réalisée par montée progressive de la température par palier de 5°C.

Dans un mode de réalisation, l'étape de séchage par dessication est réalisée par montée progressive de la température de 5°C.

Dans un mode de réalisation, l'étape de séchage par dessication est réalisée pendant une durée comprise dans un intervalle allant de 1 heure à 12 heures.

Dans un mode de réalisation, l'étape de séchage par dessication est réalisée pendant une durée comprise dans un intervalle allant de 2 heures à 10 heures.

Dans un mode de réalisation, l'étape de séchage par dessication est réalisée pendant une durée comprise dans un intervalle allant de 3 heures à 7 heures.

Dans un mode de réalisation, l'étape de séchage par dessication est réalisée pendant une durée comprise dans un intervalle allant de 4 heures à 6 heures.

Dans un mode de réalisation, l'étape de séchage par dessication est réalisée sous des pressions comprises dans un intervalle allant de 50 à 900 micro-bars (50 micro-bars ≤ pressions ≤ 900 micro-bars).

Dans un mode de réalisation, l'étape de séchage par dessication est réalisée sous des pressions comprises dans un intervalle allant de 100 à 800 micro-bars (100 micro-bars ≤ pressions ≤ 800 micro-bars).

Dans un mode de réalisation, l'étape de séchage par dessication est réalisée sous des pressions comprises dans un intervalle allant de 400 à 600 micro-bars (400 micro-bars ≤ pressions ≤ 600 micro-bars).

Dans un mode de réalisation, l'étape de séchage par dessication est réalisée sous des pressions d'environ 600 micro-bars.

Dans un mode de réalisation, l'étape de séchage par dessication est réalisée sous des pressions d'environ 400 micro-bars.

Dans un mode de réalisation, la structure placentaire et/ou ombilicale sur laquelle on applique l'étape de séchage par dessication présente un taux d'humidité avant ladite étape de séchage par dessication d'au moins 50 %.

Dans un mode de réalisation, la structure placentaire et/ou ombilicale sur laquelle on applique l'étape de séchage par dessication présente un taux d'humidité avant ladite étape de séchage par dessication d'au moins 60 %.

Dans un mode de réalisation, la structure placentaire et/ou ombilicale sur laquelle on applique l'étape de séchage par dessication présente un taux d'humidité avant ladite étape de séchage par dessication d'au moins 70 %.

Dans un mode de réalisation, la structure placentaire et/ou ombilicale sur laquelle on applique l'étape de séchage par dessication présente un taux d'humidité avant ladite étape de séchage par dessication d'au moins 80 %.

Dans un mode de réalisation, la structure placentaire et/ou ombilicale sur laquelle on applique l'étape de séchage par dessication présente un taux d'humidité avant ladite étape de séchage par dessication d'au moins 90 %.

Dans un mode de réalisation, l'étape de séchage par dessication est réalisée par montée progressive de la température jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C jusqu'à 50°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 2°C jusqu'à 45°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 4°C jusqu'à 40°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 8°C jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C jusqu'à 35°C et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape de séchage par dessication est réalisée par montée progressive de la température jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C jusqu'à 50°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 2°C jusqu'à 45°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 4°C jusqu'à 40°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 8°C jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C jusqu'à 35°C et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape de séchage par dessication est réalisée par montée progressive de la température jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C jusqu'à 50°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 2°C jusqu'à 45°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 4°C jusqu'à 40°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 8°C jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C jusqu'à 35°C et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température d'au moins 0°C à 35°C (0°C ≤ température ≤ 35°C) et sous des pressions comprises dans un intervalle allant de 400 à 600 micro-bars (400 micro-bars ≤ pressions ≤ 600 micro-bars).

Dans un mode de réalisation, l'étape de séchage par dessication est réalisée par montée progressive de la température dans un intervalle allant de 4°C à 35°C (4°C ≤ température ≤ 35°C) et sous des pressions comprises dans un intervalle allant de 400 à 600 micro-bars (400 micro-bars ≤ pressions ≤ 600 micro-bars).

Dans un mode de réalisation, la structure placentaire et/ou ombilicale sur laquelle est viro-inactivée.

Au sens de la présente invention, on entend par « viro-inactivation » une technique qui permet de réduire fortement ou totalement, et définitivement, la capacité d'action des virus. Ceux-ci, définis comme inactivés, perdent leurs capacités pathogéniques et de réplication par une diminution de leur population de 4 log lors des titrages résiduels qui suivent une ou deux étapes chimiques indépendantes, que ce soit sur des virus enveloppés, ou non enveloppés, ADN ou ARN. Cette étape permet notamment de disposer d'un produit exempt d'agents microbiologiques tels que bactéries, virus, parasites.

La viro-inactivation pourra être effectuée par toute les méthodes classiquement utilisées par l'Homme du métier.

Dans un mode de réalisation, la viro-inactivation est réalisée dans les conditions ci-dessous décrites.

Dans un mode de réalisation, la structure placentaire et/ou ombilicale est stérile.

Selon la présente invention, on entend par « stérile » une structure exempte de germe, à l'état naturel ou après stérilisation.

La stérilisation pourra être effectuée par toute les méthodes classiquement utilisées par l'Homme du métier.

Dans un mode de réalisation, la stérilisation est réalisée dans les conditions ci-dessous décrites.

Dans un mode de réalisation, la structure placentaire et/ou ombilicale est dévitalisée et/ou décellularisé.

Dans un mode de réalisation, la structure placentaire et/ou ombilicale est dévitalisée.

Dans un mode de réalisation, la structure placentaire et/ou ombilicale est décellularisé.

La présente invention a également pour objet un procédé séchage par dessication d'une structure placentaire et/ou ombilicale comprenant les étapes de :

a) On dispose d'au moins une structure placentaire et/ou ombilicale,
b) On sèche la ou les structure(s) placentaire(s) et/ou ombilicale(s) par au moins une étape de dessication,
c) On obtient une structure placentaire et/ou ombilicale séchée par dessication.

Dans un mode de réalisation, la structure placentaire et/ou membranaire dont on dispose à l'étape a) est telle que décrite ci-dessus.

Dans un mode de réalisation, la structure placentaire et/ou ombilicale dont on dispose à l'étape a) est caractérisée en ce qu'elle est d'origine animale ou humaine.

Dans un mode de réalisation, la structure placentaire et/ou ombilicale dont on dispose à l'étape a) est caractérisée en ce que la structure placentaire et/ou ombilicale est d'origine humaine.

Dans un mode de réalisation, la structure placentaire et/ou ombilicale dont on dispose à l'étape a) est obtenue selon le protocole tel que décrit dans la demande WO2017140914A1.

La structure placentaire et/ou ombilicale selon la présente invention est obtenue après l'accouchement d'un donneur proprement informé et consentant en accord avec exigences des directives Européennes et la Loi Bioéthique. Cet accouchement peut s'être déroulé par césarienne ou par voie basse.

Par exigence sanitaire relative aux dons de tissus et de cellules d'origine humaine, une qualification socio-clinique du donneur et biologique en amont du donneur est systématique. Cette qualification implique une recherche de virus HIV, hépatites B, C, HTLV et de la bactérie tréponème pâle responsable de la syphilis.

Dans un mode de réalisation, on dispose d'une structure placentaire à l'étape a).

Dans un mode de réalisation, la structure placentaire dont on dispose à l'étape a) est caractérisée en ce qu'elle comprend tout ou partie du placenta.

Dans un mode de réalisation, la structure placentaire dont on dispose à l'étape a) est un tissu placentaire.

Dans un mode de réalisation, la structure placentaire dont on dispose à l'étape a) est caractérisée qu'elle comprend tout ou partie d'au moins une couche membranaire issue de tissue placentaire.

Dans un mode de réalisation, la structure placentaire on dispose à l'étape a) est caractérisée en ce qu'elle comprend tout ou partie d'au moins une couche membranaire issue de tissus placentaires comprenant de la membrane amniotique, de la membrane choriale, de la membrane tissulaire spongieuse et/ou de la membrane chorioamniotique, chacun de ces feuillets pouvant inclure, ou non, les sous-structures physiologiques constitutives de l'amnios et/ou du chorion.

Dans un mode de réalisation, la structure placentaire dont on dispose à l'étape a) est caractérisée en ce qu'elle comprend tout ou partie d'au moins une couche membranaire issue de tissus placentaires d'origine animale ou humaine.

Dans un mode de réalisation, la structure placentaire dont on dispose à l'étape a) est caractérisée en ce qu'elle comprend tout ou partie d'au moins une couche membranaire issue de tissus placentaires d'origine humaine.

Dans un mode de réalisation, la structure placentaire dont on dispose à l'étape a) est caractérisée en ce qu'elle comprend tout ou partie d'au moins une couche membranaire issue de tissus placentaires comprenant tout ou partie de la membrane amniotique et/ou tout ou partie de la membrane choriale.

Dans un mode de réalisation, la structure placentaire dont on dispose à l'étape a) est caractérisée qu'elle comprend tout ou partie d'au moins une couche membranaire issue de tissus placentaires comprend tout ou partie de la membrane amniotique.

Dans un mode de réalisation, la structure placentaire dont on dispose à l'étape a) est caractérisée en ce qu'elle comprend de la membrane amniotique humaine.

Dans un mode de réalisation, la structure placentaire dont on dispose à l'étape a) est caractérisée en ce qu'elle comprend tout ou partie d'au moins une des sous-couches physiologiques de la membrane amniotique choisies dans le groupe comprenant la couche cellulaire épithéliale, la membrane basale, la couche compacte, la couche fibroblastique et/ou la couche spongieuse.

Dans un mode de réalisation préférentiel, la structure placentaire dont on dispose à l'étape a) est caractérisée en ce qu'elle comprend de la membrane amniotique comprenant sa couche spongieuse.

Dans un mode de réalisation, la structure placentaire dont on dispose à l'étape a) est caractérisée en ce qu'elle est constituée de la membrane amniotique comprenant sa couche spongieuse.

Dans un mode de réalisation, la structure placentaire dont on dispose à l'étape a) est caractérisée en ce qu'elle comprend au moins une couche membranaire issue de tissus placentaires comprenant tout ou partie de la membrane choriale.

Dans un mode de réalisation, la structure placentaire dont on dispose à l'étape a) est caractérisée en ce qu'elle comprend au moins une les sous-couches physiologiques de la membrane choriale choisi dans le groupe comprenant la couche réticulaire, la membrane basale et/ou la couche cellulaire trophoblastique.

Dans un mode de réalisation, on dispose d'une structure ombilicale à l'étape a).

Dans un mode de réalisation, la structure ombilicale dont on dispose à l'étape a) est caractérisée en ce qu'elle comprend tout ou partie du cordon ombilical.

Dans un mode de réalisation, la structure ombilicale dont on dispose à l'étape a) est un tissu ombilical.

Dans un mode de réalisation, la structure ombilicale dont on dispose à l'étape a) est caractérisée en ce qu'elle comprend de la gelée de Wharton, au moins un vaisseau ombilical et/ou de la membrane amniotique de cordon ombilical.

Dans un mode de réalisation, la structure ombilicale dont on dispose à l'étape a) est caractérisée en ce qu'elle comprend de la gelée de Wharton.

Dans un mode de réalisation, la structure ombilicale dont on dispose à l'étape a) est caractérisée en ce qu'elle comprend au moins un vaisseau ombilical.

Dans un mode de réalisation, la structure ombilicale dont on dispose à l'étape a) est caractérisée en ce qu'elle comprend au moins un vaisseau ombilical choisi dans le groupe comprenant une veine ombilicale et/ou au moins une artère ombilicale.

Dans un mode de réalisation, la structure ombilicale dont on dispose à l'étape a) est caractérisée en ce qu'elle comprend de la membrane amniotique de cordon ombilical.

Dans un mode de réalisation, l'étape b) de séchage par dessication comprend l'application de chaleur, de vide et/ou d'une matière hygroscopique.

Dans un mode de réalisation, l'étape b) de séchage par dessication comprend l'application d'une part de la chaleur et d'autre part de vide et/ou d'une matière hygroscopique.

Dans un mode de réalisation, l'étape b) de séchage par dessication comprend l'application de chaleur et de vide.

Dans un mode de réalisation, l'étape b) de séchage par dessication ne comprend pas de congélation et/ou de surgélation de la structure placentaire et/ou ombilicale.

Dans un mode de réalisation, dans l'étape b) de séchage par dessication, la température minimale est choisie de manière à éviter la formation de cristaux d'eau en fonction de la pression appliquée.

Dans un mode de réalisation, l'étape b) de séchage par dessication est réalisée par montée progressive de la température à partir d'au moins 0°C (0°C ≤ température).

Dans un mode de réalisation, l'étape b) de séchage par dessication est réalisée par montée progressive de la température à partir d'au moins 2°C (2°C ≤ température).

Dans un mode de réalisation, l'étape b) de séchage par dessication est réalisée par montée progressive de la température à partir d'au moins 4°C (4°C ≤ température).

Dans un mode de réalisation, l'étape b) de séchage par dessication est réalisée par montée progressive de la température jusqu'à 35°C.

Dans un mode de réalisation, l'étape b) de séchage par dessication est réalisée par montée progressive de la température d'au moins 0°C jusqu'à 35°C.

Dans un mode de réalisation, l'étape b) de séchage par dessication est réalisée par montée progressive de la température d'au moins 0°C à 35°C (0°C ≤ température ≤ 35°C).

Dans un mode de réalisation, l'étape b) de séchage par dessication est réalisée par montée progressive de la température dans un intervalle allant de 4°C à 35°C (4°C ≤ température ≤ 35°C).

Dans un mode de réalisation, l'étape b) de séchage par dessication est réalisée par montée progressive de la température par paliers de 1 à 10°C (1°C ≤ température ≤ 10°C).

Dans un mode de réalisation, l'étape b) de séchage par dessication est réalisée par montée progressive de la température par palier de 1 à 10°C.

Dans un mode de réalisation, l'étape b) de séchage par dessication est réalisée par montée progressive de la température par palier de 2 à 8°C.

Dans un mode de réalisation, l'étape b) de séchage par dessication est réalisée par montée progressive de la température par palier de 3 à 7°C.

Dans un mode de réalisation, l'étape b) de séchage par dessication est réalisée par montée progressive de la température par palier de 5°C.

Dans un mode de réalisation, l'étape b) de séchage par dessication est réalisée par montée progressive de la température de 5°C.

Dans un mode de réalisation, l'étape b) de séchage par dessication est réalisée pendant une durée comprise dans un intervalle allant de 1 heure à 12 heures.

Dans un mode de réalisation, l'étape b) de séchage par dessication est réalisée pendant une durée comprise dans un intervalle allant de 2 heures à 10 heures.

Dans un mode de réalisation, l'étape b) de séchage par dessication est réalisée pendant une durée comprise dans un intervalle allant de 3 heures à 7 heures.

Dans un mode de réalisation, l'étape b) de séchage par dessication est réalisée pendant une durée comprise dans un intervalle allant de 4 heures à 6 heures.

Dans un mode de réalisation, l'étape b) de séchage par dessication est réalisée sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée sous des pressions d'environ 600 micro-bars.

Dans un mode de réalisation, l'étape b) de séchage par dessication est réalisée sous des pressions d'environ 400 micro-bars.

Dans un mode de réalisation, l'étape b) de séchage par dessication est réalisée par montée progressive de la température jusqu'à 35°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 0°C jusqu'à 50°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 2°C jusqu'à 45°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 4°C jusqu'à 40°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 8°C jusqu'à 35°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 0°C jusqu'à 35°C et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température d'au moins 0°C à 35°C (0°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température dans un intervalle allant de 4°C à 35°C (4°C ≤ température ≤ 35°C) et sous des pressions comprises dans un intervalle allant de 50 à 900 micro-bars (50 micro-bars ≤ pressions ≤ 900 micro-bars).

Dans un mode de réalisation, l'étape b) de séchage par dessication est réalisée par montée progressive de la température jusqu'à 35°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 0°C jusqu'à 50°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 2°C jusqu'à 45°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 4°C jusqu'à 40°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 8°C jusqu'à 35°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 0°C jusqu'à 35°C et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température d'au moins 0°C à 35°C (0°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température dans un intervalle allant de 4°C à 35°C (4°C ≤ température ≤ 35°C) et sous des pressions comprises dans un intervalle allant de 100 à 800 micro-bars (100 micro-bars ≤ pressions ≤ 800 micro-bars).

Dans un mode de réalisation, l'étape b) de séchage par dessication est réalisée par montée progressive de la température jusqu'à 35°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 0°C jusqu'à 50°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 2°C jusqu'à 45°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 4°C jusqu'à 40°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 8°C jusqu'à 35°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 0°C jusqu'à 35°C et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température d'au moins 0°C à 35°C (0°C ≤ température ≤ 35°C) et sous des pressions comprises dans un intervalle allant de 400 à 600 micro-bars (400 micro-bars ≤ pressions ≤ 600 micro-bars).

Dans un mode de réalisation, l'étape b) de séchage par dessication est réalisée par montée progressive de la température dans un intervalle allant de 4°C à 35°C (4°C ≤ température ≤ 35°C) et sous des pressions comprises dans un intervalle allant de 400 à 600 micro-bars (400 micro-bars ≤ pressions ≤ 600 micro-bars).

Dans un mode de réalisation, le procédé selon la présente invention ne comprend aucune étape de lyophilisation.

Dans un mode de réalisation, au moins une structure placentaire et/ou ombilicale dont on dispose à l'étape a) est préalablement congelée.

Dans un mode de réalisation, au moins une structure placentaire et/ou ombilicale dont on dispose à l'étape a) est préalablement placée dans une boîte stérile, congelée et transportée à une température de -20°C, puis stocké à -80°C.

Dans un mode de réalisation, au moins une structure placentaire et/ou ombilicale dont on dispose à l'étape a) est transporté à +4 °C.

Dans un mode de réalisation, au moins une structure placentaire et/ou ombilicale dont on dispose à l'étape a) n'a pas été congelée initialement, la découpe et la séparation de la structure placentaire et/ou ombilicale est réalisée après le transport à +4°C.

Dans un mode de réalisation, au moins une structure placentaire et/ou ombilicale dont on dispose à l'étape a) est conservée à -80°C.

Dans un mode de réalisation, le placenta non séparé de ses tissus physiologiques peut être congelé pour conservation à une température de -80°C pendant une durée allant jusqu'à 2 ans.

Dans un mode de réalisation, au moins une structure placentaire et/ou ombilicale dont on dispose à l'étape a) à l'état congelé est lavée dans de l'eau purifiée ou séjourne dans un bain d'eau purifiée.

Cette étape assure tant la décongélation de la ou des couche(s) membranaire(s) issue(s) de tissus placentaires si nécessaire jusqu'à la température ambiante, qu'une lyse des cellules présentes dans le la ou les couche(s) membranaire(s) issue(s) de tissus placentaires par effet de pression osmotique.

Dans un mode de réalisation, au moins une structure placentaire et/ou ombilicale dont on dispose à l'étape a) est viro-inactivée.

Dans un mode de réalisation, le procédé selon l'invention comprend une étape a') de viro-inactivation de la ou des structure(s) placentaire(s) et/ou ombilicale(s) suite à l'étape a).

Dans un mode de réalisation, l'étape a') de viro inactivation comprend une étape i) de viro-inactivation comprenant l'application sur la ou les structure(s) placentaire(s) et/ou ombilicale(s) d'un lavage et/ou le séjour de cette dernière dans un bain, composé d'un premier agent viro-inactivant.

Dans un mode de réalisation, le premier agent viro-inactivant est l'éthanol.

Dans un mode de réalisation, le premier agent viro-inactivant est l'éthanol à une teneur en alcool de 50% à 80% (v/v) (50%≤ teneur en alcool ≤80%).

Dans un mode de réalisation, le premier agent viro-inactivant est l'éthanol à une teneur en alcool de 70% (v/v).

Dans un mode de réalisation, l'étape i) de viro-inactivation est effectuée pendant une durée comprise dans un intervalle allant de 30 minutes à 120 minutes (30 min≤ durée ≤120 min).

Dans un mode de réalisation, l'étape i) de viro-inactivation est effectuée pendant une durée comprise dans un intervalle allant de 45 minutes à 100 minutes (45 min≤ durée ≤100 min).

Dans un mode de réalisation, l'étape i) de viro-inactivation est effectuée pendant une durée d'environ 60 minutes.

Dans un mode de réalisation, l'étape i) de viro-inactivation est effectuée en traitant la ou les structure(s) placentaire(s) et/ou ombilicale(s) avec de l'éthanol à une teneur en alcool de 70% (v/v) pendant une durée d'environ 60 minutes.

Dans un mode de réalisation, l'étape i) est suivi d'une étape i') de lavage de la ou des structure(s) placentaire(s) et/ou ombilicale(s) par de l'eau purifiée ou un séjour dans un bain d'eau purifiée.

Dans un mode de réalisation, l'étape a') de viro inactivation comprend une seconde étape ii) de viro inactivation consécutive à l'étape i) ou i') comprenant l'application à la ou aux structure(s) placentaire(s) et/ou ombilicale(s) d'un lavage et/ou le séjour de cette dernière dans un bain, composé d'un deuxième agent de viro-inactivation.

Dans un mode de réalisation, le deuxième agent de viro inactivation est le peroxyde d'hydrogène.

Dans un mode de réalisation, le deuxième agent de viro-inactivation est le peroxyde d'hydrogène sous une forme choisie parmi une solution aqueuse, et un gaz.

Dans un mode de réalisation, le deuxième agent de viro-inactivation est le peroxyde d'hydrogène sous forme de solution aqueuse dans une concentration comprise dans un intervalle allant de 1% à 30% (w/v) (1% ≤ concentration w/v ≤ 30%).

Dans un mode de réalisation, le deuxième agent de viro-inactivation est le peroxyde d'hydrogène sous forme de solution aqueuse dans une concentration comprise dans un intervalle allant de 3% à 10% (w/v) (3% ≤ concentration w/v ≤ 10%).

Dans un mode de réalisation, l'étape ii) de viro-inactivation est effectuée pendant une durée comprise dans un intervalle allant de 30 minutes à 120 minutes (30 mn≤ durée ≤120 mn).

Dans un mode de réalisation, l'étape ii) de viro-inactivation est effectuée pendant une durée comprise dans un intervalle allant de 45 à 100 minutes (45 mn≤ durée ≤100 mn).

Dans un mode de réalisation, l'étape ii) de viro-inactivation est effectuée pendant une durée d'environ 60 minutes.

Dans un mode de réalisation, le procédé est caractérisé en ce que la deuxième étape de viro-inactivation ii) de la ou des structure(s) placentaire(s) et/ou ombilicale(s) comprend deux sous-étapes de traitement par le deuxième agent de viro-inactivation choisi dans la famille des peroxydes.

Au sens de la présente invention, on entend par « peroxydes » tout composé chimique contenant un groupe fonctionnel de formule générale R-O-O-R' (deux atomes d'oxygène voisins liés entre eux) où R et R' sont des atomes d'hydrogène ou des radicaux alkyls quelconques.

Selon un mode de réalisation, la deuxième étape de viro-inactivation ii) de la ou des structure(s) placentaire(s) et/ou ombilicale(s) comprend deux sous-étapes de traitement par le peroxyde d'hydrogène :

- une première sous-étape de traitement ii') s'effectuant avec une solution de peroxyde d'hydrogène à une concentration supérieure à 10% w/v pendant une durée d'au moins de 20 minutes ; et
- une deuxième sous-étape de traitement ii") s'effectuant avec une solution de peroxyde d'hydrogène à une concentration inférieure à 5 % w/v pendant une durée supérieure à 50 minutes.

Selon un autre mode de réalisation, la deuxième étape de viro-inactivation ii) la ou des structure(s) placentaire(s) et/ou ombilicale(s) comprend deux sous-étapes de traitement par le peroxyde d'hydrogène :

- une première sous-étape de traitement ii') s'effectuant avec une solution de peroxyde d'hydrogène à une concentration de 30% w/v pendant une durée d'environ 15 minutes ; et
- une deuxième sous-étape de traitement ii')' s'effectuant avec une solution de peroxyde d'hydrogène à une concentration de 3% w/v pendant une durée d'environ 60 minutes.

Dans un mode de réalisation, l'étape a') de viro inactivation comprend une troisième étape iii) consécutive à l'étape ii) comprenant au moins une étape de neutralisation, par application à la ou aux structure(s) placentaire(s) et/ou ombilicale(s) comprend d'un lavage et/ ou d'un séjour de cette dernière dans un bain, composé d'au moins un tampon basique.

Dans un mode de réalisation, la troisième étape iii) comprend deux bains effectués dans un tampon basique contenant de l'hydroxyde de sodium dilué et dont le pH est ajusté à une valeur comprise dans un intervalle allant de 7 à 10 (7≤pH ≤10).

Dans un mode de réalisation, la troisième étape iii) comprend deux bains effectués dans un tampon basique contenant de l'hydroxyde de sodium dilué et dont le pH est ajusté à une valeur comprise dans un intervalle allant de 8 à 9 (8≤pH ≤9).

Dans un mode de réalisation, la troisième étape iii) comprend deux bains effectués dans un tampon basique contenant de l'hydroxyde de sodium dilué et dont le pH est ajusté à une valeur 8,5.

Dans un mode de réalisation, l'étape a') de viro inactivation comprend une dernière étape iv) comprenant l'application à la ou aux structure(s) placentaire(s) et/ou ombilicale(s) comprend d'un lavage et/ou d'un séjour dans un bain, composé d'au moins une solution tampon assurant un rééquilibrage physiologique.

Dans un mode de réalisation, la dernière étape iv) comprend deux bains effectués en solution de PBS.

Dans un mode de réalisation, les étapes de décongélation, viro-inactivation, lavage, ajustage de pH et mise en tampon s'effectuent à température ambiante.

Dans un mode de réalisation, le procédé selon l'invention comprend en outre une dernière étape de stérilisation de la ou des structure(s) placentaire(s) et/ou ombilicale(s) séchée(s) par dessication obtenue à l'étape c).

La stérilisation pourra être réalisé par toute méthode classiquement connue de l'Homme du métier.

Dans un mode de réalisation, la stérilisation est réalisée par irradiation.

Dans un mode de réalisation, la stérilisation est réalisée par irradiation par des rayonnement gamma.

Dans un mode de réalisation, la stérilisation est réalisée par irradiation par des rayonnements gamma à une dose comprise dans un intervalle allant de 25 à 35 kGrays (25 kGrays ≤ dose ≤ 35 kGrays).

Dans un mode de réalisation, l'un quelconque des procédés selon l'invention comprend une étape préalable de séparation de la ou des couche(s) membranaire(s) issue(s) de tissus placentaires.

Dans les exemples, tous les pourcentages sont donnés en poids sauf indication contraire, la température est exprimée en degré Celsius sauf indication contraire, les essais sont réalisés à température ambiante sauf indication contraire, et la pression est la pression atmosphérique sauf indication contraire.

## EXEMPLES

### Exemple 1 : Procédé de préparation.

### Exemple 1.a. Exemple de procédé de préparation d'une structure placentaire consistant en une couche membranaire.

Un donneur proprement informé et consentant en accord avec les exigences de la Déclaration d'Helsinki offre en don le tissu placentaire issu d'un accouchement.

Le tissu placentaire est récupéré en salle d'accouchement. Il est congelé à une température de -20°C.

Au laboratoire, en salle stérile, la procédure suivante est appliquée :

En salle stérile, la membrane amniotique est isolée et est nettoyée.

La membrane amniotique subit une succession de bains assurant la viro inactivation de celle-ci. Une agitation douce à environ 30 rpm du milieu liquide est appliquée lors de chaque bain

Dans un premier temps, la membrane amniotique est déposée dans un bain d'eau purifiée à température ambiante pendant environ 3 heures.

Puis, la membrane amniotique est transférée dans un bain décontaminant composé d'éthanol 70% v/v à température ambiante pendant environ 1 heure.

Un lavage est effectué dans de l'eau purifiée pendant environ 15 minutes à température ambiante pour retirer l'éthanol.

Afin d'assurer la seconde étape de traitement décontaminant, la membrane amniotique est transférée dans un bain composé de peroxyde d'hydrogène à 30% w/v à température ambiante pendant environ 15 minutes.

Puis la membrane amniotique est transférée dans un bain décontaminant composé de peroxyde d'hydrogène à 3% w/v à température ambiante pendant environ 1 heure.

L'action chimique appliquée à la membrane amniotique est neutralisée dans deux bains comprenant de l'hydroxyde de sodium dilué autour d'un pH de 8,5. Les bains de neutralisation s'effectuent à température ambiante pendant environ 15 minutes.

Afin d'assurer un rééquilibrage du pH et pour éliminer au mieux les résidus organiques se détachant du tissu d'intérêt, la membrane amniotique est transférée dans deux bains successifs de tampon PBS à température ambiante pendant environ 15 minutes afin d'assurer son rééquilibrage physiologique.

Enfin, la membrane amniotique est transférée dans un dernier bain à l'eau purifiée, à température ambiante, pendant 30 minutes.

On obtient une membrane amniotique obtenue selon ce traitement chimique est désinfectée et viro-inactivée.

**Exemple 1.b : Exemple de réalisation d'une structure placentaire consistant en une couche membranaire séchée par au moins une étape de dessication selon l'invention.**

Une structure placentaire consistant en une couche membranaire est préparée selon le protocole tel que décrit à l'exemple 1a) et soumise à une étape de séchage par dessication dans les conditions suivantes.

a) Température de 8,0°C pendant 5 minutes,
b) Température de 8,0° pendant 120 minutes,
c) Température de 10,0° pendant 30 minutes sous une pression sous vide de 400 μbar,
d) Température de 15,0°C pendant 30 minutes,
e) Température de 20,0°C pendant 30 minutes,
f) Température de dessication : 1,0°C,
g) Température de 25,0°C pendant 30 minutes,
h) Température de 30,0°C pendant 30 minutes,
i) Température de 35,0°C pendant 30 minutes,
j) Fin du cycle par un retour à l'atmosphère normale et à la température ambiante pendant une durée de 5 minutes.

La structure obtenue est photographiée.

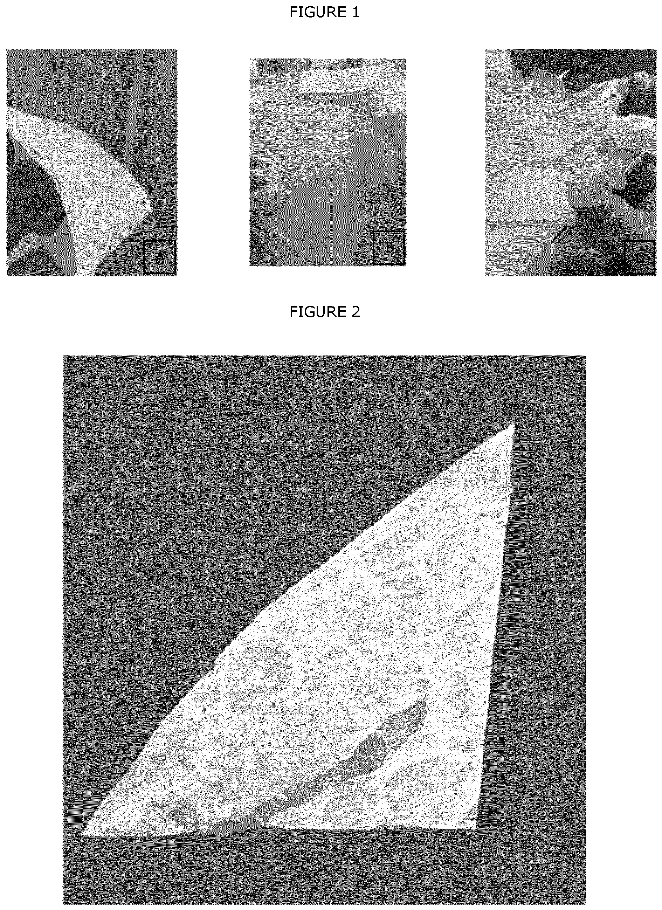

La structure obtenue est présentée sur l'image A de la Figure 1.

**Exemple 1.b bis: Exemple de réalisation d'une structure placentaire consistant en une couche membranaire séchée par au moins une étape de dessication selon l'invention.**

Une structure placentaire consistant en une couche membranaire est préparée selon le protocole tel que décrit à l'exemple 1a) et soumise à une étape de séchage par dessication dans les conditions suivantes.

a) Température de 8,0°C pendant 5 minutes,
b) Température de 8,0° pendant 120 minutes,
c) Température de 10,0° pendant 30 minutes sous une pression sous vide de 400 μbar,
d) Température de 15,0°C pendant 30 minutes sous une pression sous vide de 400 μbar,
e) Température de 20,0°C pendant 30 minutes sous une pression sous vide de 400 μbar,
f) Température de 25,0°C pendant 30 minutes sous une pression sous vide de 200 μbar,
g) Température de 30,0°C pendant 30 minutes sous une pression sous vide de 200 μbar,
h) Température de 35,0°C pendant 30 minutes sous une pression sous vide de 200 μbar,
i) Fin du cycle par un retour à l'atmosphère normale et à la température ambiante pendant une durée de 5 minutes.

**Exemple 1c : Exemple comparatif de préparation d'une structure placentaire consistant en une couche membranaire séchée par lyophilisation.**

Une couche membranaire issue de tissus placentaires est préparée selon le protocole tel que décrit à l'exemple 1a).

Elle est récupérée et soumise à un séchage par lyophilisation dans les conditions suivantes.

a) Température de - 10,0°C pendant 5 minutes,
b) Température de - 15,0°C pendant 90 minutes,
c) Température de - 45,0°C pendant 5 minutes,
d) Température de - 45,0°C pendant 90 minutes,
e) Température de 10,0°C pendant 180 minutes sous une pression sous vide de 200 μbar,
f) Température de congélation -40°C,
g) Mise sous vide à une pression de 300 μbar,
h) Température de 10,0°C pendant 210 minutes sous une pression sous vide de 200 μbar,
i) Température de 25,0°C pendant 30 minutes sous une pression sous vide de 200 μbar,
j) Température de 25,0°C pendant 120 minutes sous une pression sous vide de 200 μbar,
k) Température de 35,0°C pendant 60 minutes sous une pression sous vide de 50 μbar,

l) Température de 35,0°C pendant 90 minutes sous une pression sous vide de 50 μbar,

m) Température de 25,0°C pendant 40 minutes sous une pression sous vide de 50 μbar,

n) Température de 25,0°C pendant 20 minutes sous une pression sous vide de 50 μbar,

o) Fin du cycle par un retour à l'atmosphère normale et à la température ambiante pendant une durée de 5 minutes.

La structure obtenue est photographiée. Cette photographie est présentée sur l'image B de la Figure 1.

**Exemple 1d : Exemple comparatif de préparation d'une structure placentaire consistant en une couche membranaire séchée par séchage à l'air libre.**

Une couche membranaire issue de tissus placentaires est préparée selon le protocole tel que décrit à l'exemple 1a).

La couche membranaire est séchée à température ambiante pendant 1 à 2 heures puis photographiée.

La structure obtenue est présentée sur l'image C de la Figure 1.

**Exemple 2 : Comparaison visuelle entre les structures placentaires consistant en des couches membranaires séchées par différentes méthodes.**

En comparant les structures obtenues (voir Figure 1) on observe clairement que la couche membranaire issue de tissus placentaires séchée par au moins une étape de séchage par dessication (image A) est visuellement moins transparente donc plus épaisse, se plie plus naturellement et est moins collante au toucher que la couche membranaire séchée par au moins une étape de lyophilisation (image B). La couche membranaire séchée à l'air libre (image C) est la plus rigide et celle qui présente le plus de marques donnant une apparence froissée et la plus transparente donc la plus fine.

**Exemple 3 : Comparaison de la souplesse entre une couche membranaire issue de tissus placentaires séchée par au moins une étape de séchage par dessication et une couche membranaire issue de tissus placentaires séchée par au moins une étape de lyophilisation.**

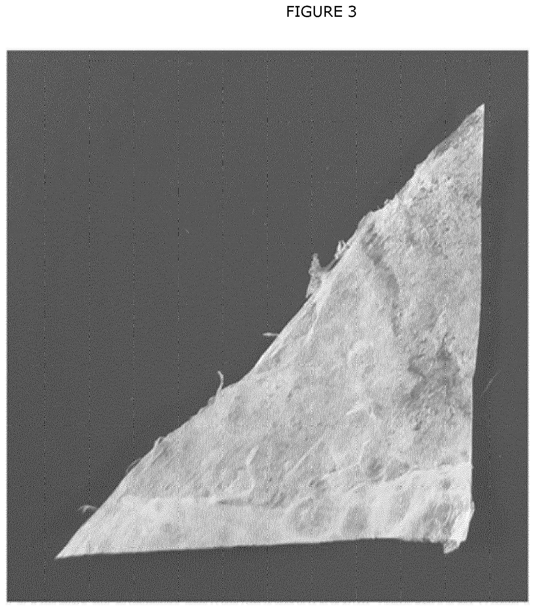

La souplesse est évaluée en pliant chacune des couches membranaires obtenues selon le procédé décrit aux exemples 1b et 1c sur elles-mêmes dans le sens de la diagonale. Les résultats sont présentés en Figure 2 pour la couche membranaire issue de tissus placentaires séchée par au moins une étape de séchage par dessication et en Figure 3 pour la couche membranaire issue de tissus placentaires séchée par au moins une étape de lyophilisation.

On observe clairement que la couche membranaire ayant subi une étape de lyophilisation se replie sur elle-même avec un bord moins arrondi que la couche membranaire dessiquée. En appuyant sur le bord on ressent la possibilité d'une cassure en cas d'appui trop important.

La couche membranaire ayant subi une étape de séchage par dessication est donc plus souple est moins cassante que la membrane amniotique ayant subi une étape de lyophilisation.

**Exemple 4 : Comparaison de l'épaisseur moyenne entre une couche membranaire issue de tissus placentaires séchée par au moins une étape de séchage par dessiccation et une couche membranaire issue de tissus placentaires séchée lyophilisation.**

Les couches membranaires obtenues selon le procédé tel que décrit dans l'exemple 1b et 1c sont découpées à l'aide d'un scalpel en éléments de type filaire, plus précisément en bandelettes, d'une largeur d'environ 0,02 mm.

Les bandelettes sont placées verticalement sur une lame et maintenues avec une pince fine en inox dont la taille est préalablement mesurée à l'aide d'un pied à coulisse.

L'ensemble est disposé sous un microscope (Nikon, Eclipse E200) avec un grossissement total de x40 entre l'objectif et l'oculaire. Des photographies sont réalisées pour 4 bandelettes de couches membranaires ayant subi une étape de séchage par dessication selon le protocole tel que décrit dans l'exemple 1b et pour 4 bandelettes de couche membranaire ayant subi une étape de séchage par lyophilisation selon le protocole tel que décrit dans l'exemple 1c.

L'épaisseur des bandelettes et de la pince en inox sont mesurées au pied à coulisse sur les photographies imprimées.

L'épaisseur réelle des bandelettes est calculée à l'aide de la formule suivante :

$$Epaisseur\ réelle\ (mm) = Epaisseur\ sur\ la\ photo\ (mm) \times \frac{Largeur\ réelle\ de\ la\ pince\ (mm)}{Largeur\ de\ la\ pince\ sur\ la\ photo\ (mm)}$$

Des calculs de moyenne et d'écart-type sont réalisés sur un fichier Excel pour déterminer les épaisseurs moyennes des couches membranaires étudiées.

Les résultats sont exposés ci-dessous.

Tableau 1

|  | Couche membranaire dessiquée selon l'exemple 1b | Couche membranaire lyophilisée selon l'exemple 1c |
|---|---|---|
| Moyenne | 0,041 | 0,028 |
| Ecart-type | 0,003 | 0,006 |

La membrane amniotique ayant subi une étape de séchage par dessication est donc près de 50% plus épaisse que la membrane amniotique ayant subi une étape de lyophilisation.

**Exemple 5 : Comparaison du taux d'humidité moyen entre les couches membranaires issues de tissus placentaires séchées par différentes méthodes.**

Les paramètres suivants sont rentrés dans un dessiccateur infrarouge (Radwag Mac 50) : Standard -/- ; 125°C ± 2°C ; programme 5 : variation de poids inférieure à 1 mg/ 120 s.

Une fois le programme démarré, la coupelle est positionnée dans le dessiccateur et tarée.

Environ 50 mg de chaque couche membranaire obtenue selon les exemples 1b, 1c, ou 1d sont déposés dans la coupelle.

La valeur de l'humidité du produit est calculée par le dessiccateur.

Les résultats sont exposés ci-dessous.

Tableau 2

| Échantillons | Couche membranaire dessiquée selon l'exemple 1b | Couche membranaire lyophilisée selon l'exemple 1c | Couche membranaire séchage à l'air selon l'exemple 1d |
|---|---|---|---|
| Humidité (%) | 23,0277 | 12,8874 | < 10,0 |

La couche membranaire dessiquée selon le procédé de l'invention présente une humidité résiduelle près de deux fois supérieure à celle de la couche membranaire ayant subi une étape de lyophilisation et est plus de deux fois supérieur à celle de la couche membranaire séchée à l'air libre.

**Revendications**

1. Structure placentaire et/ou ombilicale **caractérisée en ce qu'**elle est séchée par au moins une étape de dessication.

2. Structure placentaire et/ou ombilicale selon la revendication 1, **caractérisée en ce que** la structure placentaire et/ou ombilicale est d'origine humaine.

3. Structure placentaire et/ou ombilicale selon l'une quelconque des revendications précédente, **caractérisée en ce que** la structure placentaire comprend tout ou partie d'au moins une couche membranaire issue de tissu placentaire.

4. Structure placentaire et/ou ombilicale selon l'une quelconque des revendications précédente, **caractérisée en ce que** la structure ombilicale comprend de la gelée de Wharton, au moins un vaisseau ombilcal et/ou de la membrane amniotique de cordon ombilical.

5. Structure placentaire et/ou ombilicale selon l'une quelconque des revendications précédente, **caractérisée en ce que** l'étape de séchage par dessication comprend d'une part de la chaleur et d'autre part de vide et/ou d'une matière hygroscopique.

6. Procédé séchage par dessication d'une structure placentaire et/ou ombilicale comprenant les étapes de :

a) On dispose d'au moins une structure placentaire et/ou ombilicale,

b) On sèche par dessication la ou les structure(s) placentaire(s) et/ou ombilicale(s),

c) On obtient une structure placentaire et/ou ombilicale séchée par dessication.

7. Procédé séchage par dessication d'une structure placentaire et/ou ombilicale selon la revendication 6, **caractérisée en ce que** la structure placentaire et/ou ombilicale est telle que définie aux revendications 2 à 4

8. Procédé séchage par dessication d'une structure placentaire et/ou ombilicale selon l'une quelconque des revendications 6 ou 7, **caractérisée en ce que** l'étape b) de séchage par dessication comprend l'application d'une part de la chaleur et d'autre part de vide et/ou d'une matière hygroscopique.

9. Procédé séchage par dessication d'une structure placentaire et/ou ombilicale selon l'une quelconque des revendications 6 à 8, **caractérisée en ce que** l'étape b) de séchage par dessication est réalisée par montée progressive de la température d'au moins 0°C jusqu'à 35°C.

10. Procédé séchage par dessication d'une structure placentaire et/ou ombilicale selon l'une quelconque des revendications 6 à 9, **caractérisée en ce que** l'étape b) de séchage par dessication est réalisée sous des pressions comprises dans un intervalle allant de 50 à 900 micro-bars.

FIGURE 1

FIGURE 2

FIGURE 3

**Europäisches Patentamt**
**European Patent Office**
**Office européen des brevets**

# RAPPORT DE RECHERCHE EUROPEENNE

Numéro de la demande

EP 24 15 5952

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X,D | GRÉMARE AGATHE ET AL: "Development of a vascular substitute produced by weaving yarn made from human amniotic membrane", BIOFABRICATION , vol. 14, no. 4 1 octobre 2022 (2022-10-01), page 045010, XP093107411, UK ISSN: 1758-5082, DOI: 10.1088/1758-5090/ac84ae Extrait de l'Internet: URL:https://iopscience.iop.org/article/10.1088/1758-5090/ac84ae/pdf [extrait le 2023-11-30] * le document en entier * ----- | 1-10 | INV. A61L27/36 A61L27/50 |
| A | US 2008/046095 A1 (DANIEL JOHN [US]) 21 février 2008 (2008-02-21) * revendications 1-20 * ----- | 1-10 | |

**DOMAINES TECHNIQUES RECHERCHES (IPC)**

A61L

Le présent rapport a été établi pour toutes les revendications

| Lieu de la recherche | Date d'achèvement de la recherche | Examinateur |
|---|---|---|
| La Haye | 15 février 2024 | Burtan, M |

EPO FORM 1503 03.82 (P04C02)

**ANNEXE AU RAPPORT DE RECHERCHE EUROPEENNE
RELATIF A LA DEMANDE DE BREVET EUROPEEN NO.**

EP 24 15 5952

La présente annexe indique les membres de la famille de brevets relatifs aux documents brevets cités dans le rapport de recherche européenne visé ci-dessus.
Lesdits members sont contenus au fichier informatique de l'Office européen des brevets à la date du
Les renseignements fournis sont donnés à titre indicatif et n'engagent pas la responsabilité de l'Office européen des brevets.

15-02-2024

| Document brevet cité au rapport de recherche | Date de publication | Membre(s) de la famille de brevet(s) | Date de publication |
|---|---|---|---|
| US 2008046095 A1 | 21-02-2008 | US 2008046095 A1 | 21-02-2008 |
| | | US 2012294810 A1 | 22-11-2012 |
| | | US 2012294811 A1 | 22-11-2012 |
| | | US 2013006385 A1 | 03-01-2013 |
| | | US 2013317625 A1 | 28-11-2013 |
| | | US 2013337035 A1 | 19-12-2013 |
| | | US 2014283990 A1 | 25-09-2014 |
| | | US 2014330391 A1 | 06-11-2014 |
| | | US 2014370068 A1 | 18-12-2014 |
| | | US 2015110850 A1 | 23-04-2015 |
| | | US 2015174297 A1 | 25-06-2015 |
| | | US 2015182661 A1 | 02-07-2015 |
| | | US 2015209475 A1 | 30-07-2015 |
| | | US 2015265747 A1 | 24-09-2015 |
| | | US 2016030634 A1 | 04-02-2016 |
| | | US 2017157295 A1 | 08-06-2017 |
| | | US 2017319628 A1 | 09-11-2017 |
| | | US 2023201419 A1 | 29-06-2023 |

EPO FORM P0460

Pour tout renseignement concernant cette annexe : voir Journal Officiel de l'Office européen des brevets, No.12/82

## RÉFÉRENCES CITÉES DANS LA DESCRIPTION

**Documents brevets cités dans la description**

- WO 2017140914 A
- WO 2017140914 A1